# EUROPEAN PATENT SPECIFICATION

(11) **EP 2 670 715 B1**
(45) Date of publication and mention of the grant of the patent: **26.04.2017**
(21) Application number: 11701285.6
(22) Date of filing: 31.01.2011
(51) Int. Cl.: C02F 11/04, C02F 3/28, C02F 3/30, C02F 3/00, C05F 17/00, C12M 1/107, C12M 1/16, C12P 5/02, C12M 1/00, C02F 101/10, C02F 101/16, C02F 101/20, C02F 103/20, C02F 103/26

(54) **INSTALLATION AND METHOD FOR BIOMASS CONVERSION INTO METHANE**
ANLAGE UND VERFAHREN ZUR UMWANDLUNG VON BIOMASSE IN METHAN
INSTALLATION ET PROCÉDÉ POUR LA CONVERSION DE BIOMASSE EN MÉTHANE

(43) Date of publication of application: 11.12.2013
(73) Proprietor: Red Patent B.V., 7213 AN Gorssel (NL)
(72) Inventor: HAZEWINKEL, Jacob Hendrik Obbo, NL-2723 BG Zoetermeer (NL)
(74) Representative: Octrooibureau Van der Lely N.V.
(86) International application number: PCT/EP2011/051308
(87) International publication number: WO 2012/103922

(56) References cited:
- EP-A1- 0 135 486
- WO-A2-2009/076947
- FR-A1- 2 924 441
- US-A- 4 213 857
- US-A1- 2010 206 791

## Description

The present invention relates to apparatuses, such as small and medium scale processing plants, for conversion of biomass into methane and other high-grade products such as fertiliser. The present invention further relates to methods and uses of the present apparatuses for conversion of biomass into methane and other high-grade products such as fertiliser.

In the Netherlands, the average annual production of biomass is approximately 30,000 tonnes dry matter of which nearly 28,000 tonnes is produced in agriculture. This tonnage represents an energy content of 475 pJ of renewable energy each year equal to 15 billion m3 natural gas. In comparison, in the Netherlands, the use of natural gas was 1400 pJ in 2008.

Part of the annual biomass production is used as marketable products, or raw materials, while the remainder is not, or scarcely, used. A large portion of the annual biomass production which is used eventually results in
organic waste streams, and especially wet organic waste streams, such as liquid manure, manure, sewage sludge, domestic vegetable waste, agricultural plant residue or domestic plant residue.

The energy content of the primarily non-used biomass and the organic waste streams of the primarily used biomass is considerable in addition to nutrient content, for example, nitrogen, phosphor, minerals and trace elements.

Conversion of non-used biomass, such as agricultural and forestry waste and organic waste streams of the primarily used biomass into high-grade products, for example natural gas, can significantly contribute to the amount of sustainable energy, or green energy, available for energy consumption and, accordingly, significantly contribute to reduction of green house gasses such as C02.

It has been estimated that 30% to 60% of the annual biomass production can be converted into methane or natural gas thereby, for example, providing a renewable potential alternative for 10% to 20% of the natural gas consumption in the Netherlands.

Conversion of organic waste streams, and especially manure, into methane has been used for decades. The most commonly used method basically comprises a large air-sealed holder in which manure is collected and allowed to ferment, i.e. convert or digest, carbon based or organic materials into methane, generally for 30 to 40 days. The resulting (bio)gas generally comprises approximately 40% C02, 60% methane and changing amounts of H₂S.

In principle, the (bio)gas produced is not directly suitable for energy consumption because, amongst others, its relatively low methane content. Additionally, the presence of large amounts of C02 and H₂S is undesired in an energy source.

In practice, it is been shown that fermentation of 25 manure alone does not suffice to efficiently convert manure into methane. The addition to the manure of additional nutrient sources such as maize is required to aid the fermentation process.

Further, in practice, it has been shown that only large scale biogas production facilities can be economically exploited for biogas production. On site small scale plants using locally produced biomass are not feasible from an investment and production yield point of view.

An additional problem associated with traditional natural gas, or methane, production using biomass is the residue obtained after fermentation. This residue comprises high concentrations of microorganisms, besides ammonia, heavy metals, phosphor and nitrogen, and is not directly suitable to be used, for example as a fertiliser, and, accordingly has to be further processed or disposed thereby, amongst others, increasing the costs of the traditional biogas production process.

A further problem especially associated with organic waste streams produced by animals, such as (liquid) manure, is the annual release of NH3, or ammonia, in the environment, for example by discarding the manure or directly using it as fertiliser. The discarded animal organic waste streams also significantly contribute to the additional direct release of methane, a green house gas, in the atmosphere.

Although the problems associated with renewable natural gas, or methane, production from biomass have been described above especially in relation to manure conversion into biogas, most of these problems, such as inefficient conversion, low-grade biogas, not feasible on a small scale, are also associated with other organic waste streams such as sewage sludge, domestic vegetable waste, agricultural plant residue or domestic plant residue.

Especially for small scale conversion of biomass, a biomass conversion plant, or installation, preferably meets most, if not all, of the requirements presented below:
- Maximal reduction of emissions of acidifying or green house gasses;
- All biomass can be processed or converted locally thus not only liquid manure;
- Recycling of phosphor, potassium and nitrogen in directly useable high grade products, for example by separating phosphor and potassium and converting NH3 into NH4N03;
- Production of biogas comprising high concentrations of methane;
- Production of stabilised organic matter, such as compost, directly useable as fertilizer;
- High energy conversion rates;
- No, or a significant reduction, of H₂S emissions;
- Fully automatable;

Especially for medium scale conversion of biomass, a biomass conversion plant or installation preferably meets most, if not all, of the requirements presented below:
- Production of biogas comprising high concentrations of methane;
- Recycling of phosphor, potassium and nitrogen in directly useable high grade products, for 20 example by separating phosphor and potassium and converting NH3 into NH4N03;
- Concentrated fertilizer liquids;
- Possibility to separate heavy metals;
- Production of substantially microbial free stabilized organic matter such as compost;
- High energy conversion rates;
- No, or a significant reduction, of emissions of acidifying, green house gasses and H₂S emissions;
- Cost efficient.

US4213857 describes a process for digestion treatment of organic waste. This waste is charged into a decomposition tank operated at a temperature of 55-75 C and a pH of 2.5 -4.0. The slurry which finished decomposition treatment is transferred to a solid liquid separator which might be a sedimentation separator. The separated liquid part obtained is charged into a mixed liquefaction tank in contact with liquefaction bacteria under anaerobic conditions below 75 C and at a pH of 4-7. The liquor after liquefaction treatment is charged into an anaerobic stirred gasification tank, and by the activities of gasification bacteria the organic acid in the liquor is converted into methane and carbon dioxide. The temperature is kept thermostatically within a range of normal to 75 C and at a pH of 7-8. The liquor that went through gasification treatment is transferred to a solid liquor separator, and separated into water and digested sludge. The digested sludge generated from this step can be disposed as fertilizer after dehydrated and dried. The separated water is put into an aerobic treatment tank for aerobic treatment. The separated water after aerobic treatment is charged into solid liquor separator, and separated to treated water and sludge.

It is an object of the present invention, amongst other objects, to provide apparatuses, or installations or plants, for conversion of biomass into methane and other high-grade products meeting at least part, if not all, of the above requirements for small scale (local) and/or medium scale (regional) production facilities of natural gas and other high-grade products.

The above object is met by an apparatus for conversion of biomass as defined by the appended claim 1, the apparatus comprises:
a) an acidification reactor (1) of a mixed fluid type reactor for microbial hydrolysis and acidification of biomass comprising at least one intake
   comprising at least one inlet for receiving biomass, at least one discharge end comprising at least one outlet for discharging acidified biomass, the acidification reactor (1) is operated at a temperature of 45°C to 70°C and a pH of 3 to 4.5;
b) a methane synthesis reactor (2) of a solid bed reactor type for the anaerobic microbial conversion of acidified biomass into methane comprising at least one intake for receiving acidified biomass from the acidification reactor (1), at least one discharge end comprising at least three outlets for discharging processed sludge biomass, methane comprising gaseous effluent and acidified liquid biomass, the methane synthesis reactor (2) is operated at a temperature of 20°C to 60°C, a pH of 6.5 to 8 and a redox potential of -150 mV to -450 mV;
c) a methane synthesis reactor (3) of a mixed fluid type reactor for anaerobic microbial conversion of acidified liquid biomass into methane comprising at least one intake comprising at least one inlet for receiving acidified liquid biomass from the methane synthesis reactor (2) and at least one discharge end comprising at least two outlets for discharging processed liquid biomass and methane comprising gaseous effluent, the methane synthesis reactor (3) is operated at a temperature of 20°C to 60°C, a pH of 6.5 to 8 and a redox potential of -150mV to -450mV;
d) a nitrification reactor (4) for aerobic microbial conversion of NH4+ into N03 comprising at least one intake comprising at least one inlet for receiving processed liquid biomass from the methane synthesis reactor (3), at least one discharge end comprising at least one outlet for discharging nitrified processed liquid biomass, the nitrification reactor (4) is operated at a temperature of 15°C to 37°C, a pH of 6.5 to 7.5 and a redox potential of more than 50 mV, characterized in that
the apparatus further comprises:
f) a composting reactor (6) for microbial decomposition of processed sludge biomass comprising at least one intake comprising at least one inlet for receiving processed sludge biomass from the methane synthesis reactor (2) and at least one discharge end comprising at least one outlet for discharging composted biomass, the composting reactor (6) is configured to be operated at a temperature of 45°C to 75°C, a pH and an oxygen concentration of 2 to 20%, wherein the composting reactor (6) comprises a further outlet for discharging acetate comprising leachate and the acidification reactor (1) comprises a further inlet for receiving the acetate comprising leachate from the composting reactor (6).

The present inventors have surprisingly found that the above combination and order of separate reactors operated under the conditions specified, provides:
- biogas comprising high concentrations of methane (approximately 60%). Because of the composition of the biogas produced (relatively free from interfering contaminants), the biogas can be easily further processed into biogas comprising 90 to 99.8% methane, for example using standard techniques such as a potassium carbonate wash or cryogenic distillation. Further, the present biogas produced comprises less than 2ppm H₂S;
- the high-grade fertilisers are produced, i.e., nitrified processed liquid biomass and processed sludge biomass, having a pH of around 7 and not comprising gaseous organic matter allowing them to be directly used as fertilizer.

The present reactors (1) to (4) are based on microbial conversion, or processing, of biomass. The microorganisms, such as fungi and bacteria, used in the reactors can be provided by, or present in, the biomass itself, or can be inoculated in the reactors at, for example, start-up of the apparatus. Suitable inoculation cultures can be found in waste and surface water purification installations.

According to the present invention, selection of species of microorganisms is not particularly important. The reaction conditions defined allow the creation of specific environments favouring the growth and/or phenotype of acid producing microorganisms, such as fungi, in the acidification reactor (1), production of methane, for example by bacteria, in the methane synthesis reactors (2) and (3) and the nitrification in the nitrification reactor (4).

The present acidification reactor (1) of a mixed fluid type reactor substantially provides acidification by acid secretion of microorganisms. However, for example, when the biomass supplied comprises a high nitrogen content, the indicated pH range can be optionally maintained by adding additional sugar or acid to the biomass or into the reactor (1).

The present inventors have surprisingly found that by microbial acidification of the biomass under the conditions specified:
- a phase separation occurs between an acidified liquid biomass comprising dissolved minerals and dissolved organic compounds such as acetic acid and hydrocarbon breakdown products, and acidified sludge biomass comprising, for example, fibre-like materials and minerals such as sand and clay;
- a surface layer is formed substantially comprised of plastics and/or lignocelluloses.

If present, contaminants such as plastics can be readily removed from the process stream by separation, or isolation, of the surface layer.

The above phase separation allows separating sludge and liquid process flows using traditional techniques such as sedimentation, filtration, tilted plate separators, or crossflow microfiltration. Additionally, separated sludge and liquid flows prevent clogging of the apparatus and allow efficient heat-exchange providing a reduction of external heat required by 60% to 70%.

After acidification of the biomass, the acidified biomass is transported to and discharged in a methane synthesis reactor (2) allowing separation of the acidified biomass in a sludge and liquid stream. The acidified sludge biomass is subjected to an anaerobic environment allowing microbial methane production and the acidified liquid biomass is discharged into a methane synthesis reactor (3) where it is separately subjected to a similar anaerobic environment allowing microbial methane production.

Thereafter, the processed liquid biomass is transported to and discharged in a nitrification reactor (4). Under the conditions specified, the nitrification reactor (4) microbially converts NH4+ (NH3) into non-gaseous N03 - thereby lowering the pH of the processed liquid biomass to a pH of 6.5 to 7.5 resulting in a directly useable, for example as a liquid fertilizer solution, neutral mixture of ammonium nitrate and urea.

The present inventors have surprisingly found that the apparatus as described above allows conversion of biomass in 1 to 2 days, in comparison, the traditional plants require 30 to 40 days, with an efficiency of conversion of 80 to 85% per day or more.

Without being limiting to the invention because of an underlying mechanism, at least a substantial part of the efficiency of the present apparatus with respect to methane production appears to be attributable to high concentrations of acetic acid in the acidified liquid biomass.

According to a preferred embodiment of the present invention, the present acidification reactor (1) further comprises an outlet for discharging H₂S comprising gaseous effluent and the apparatus further comprises:
e)an effluent gas conversion reactor (5) for aerobic microbial conversion of H₂S into S042- comprising at least one intake comprising at least inlet for receiving H₂S comprising gaseous effluent from the acidification reactor (1) and at least one discharge end comprising at least one outlet for discharging C02, H20, S042-' the effluent gas conversion reactor (5) is operated at a temperature of 15°C to 35°C, a pH of 3.0 to 4.5 and a redox potential of more than 50 mV;

The present inventors have surprisingly found that the gaseous effluent of the acidification reactor (1) substantially comprises a substantial amount of, if not all, sulphur in the form of H₂S present in the biomass supplied. Accordingly, substantially all sulphur, or at least a significant portion thereof, can be conveniently removed in a early stage of the conversion process by discharging the gaseous effluent from the acidification reactor (1).

By transporting and discharging the gaseous effluent in the present effluent gas conversion reactor (5) and subjecting it to the condition specified, microbial conversion of gaseous H₂S into S04- salts is obtained.

Acidic liquid comprising S04- discharged from the effluent gas conversion reactor (5) can be conveniently used in the apparatus for pH regulation.

The present composting reactor (6) receives processed sludge biomass from the methane synthesis reactor (2) and subjects the sludge to the indicated conditions for a period of time sufficient for drying and further digestion, such as for 10 to 30 days.

The controlled oxygen pressure and relatively high temperature ensures efficient composition. In addition, at least partially performing the process at temperatures above 70°C allows for decontaminating the compost of most potential pathogenic microorganisms.

Since the input stream of the composting reactor (6) is low in sulphur, sulphur is removed in the acidification reactor (1), but high in minerals and trace elements, the resulting composted biomass is a high-grade directly usable fertiliser.

If present in the biomass, heavy metals can be readily removed by subjecting the acidified or processed sludge biomass to a sedimentation step and removing the sediment comprising heavy metals from the process stream(s).

Acetate or acetic acid comprising leachate is produced in the composting reactor (6) as a hydrolyzation product of cellulose. Because of the relatively mild acidic nature of acetic acid, in addition to its buffering capacities, the leachate produced by the composting reactor (6) can be transported to, and discharged in, the acidification reactor (1) thereby assisting in maintaining the pH in the required range.

Gaseous effluent from the composting reactor (6) comprising NH3 can be conveniently processed in the nitrification reactor (4).

According to still another preferred embodiment, the present invention relates to an apparatus wherein the methane synthesis reactor (2) comprises a phase separation device for separating the acidified biomass into an acidified liquid biomass and an acidified sludge biomass comprising at least one inlet for receiving acidified biomass and at least two outlets for discharging acidified liquid biomass to the methane synthesis reactor (3) and acidified sludge biomass and a methane synthesis device comprising at least one inlet for receiving the acidified sludge biomass and at least one outlet for discharging processed sludge biomass, the methane synthesis device is operated at a temperature of 20°C to 60°C, a pH of 6.5 to 8 and a redox potential of -150mV to -450mV.

As indicated, the acidification of the biomass yields, amongst others, phase separation of the acidified biomass. Accordingly, in a preferred embodiment, phase separation of acidified liquid and sludge biomass is performed before methane synthesis by microbial conversion.

According to a further preferred embodiment, the present apparatus comprises between the methane synthesis reactor (3) and the nitrification reactor (4) a vacuum device for concentrating the processed liquid biomass, the vacuum device comprises an inlet for receiving processed liquid biomass from the methane synthesis reactor (3) and an outlet for discharging concentrated processed liquid biomass to the nitrification reactor (4), an outlet for discharging C02 and methane and an outlet for discharging CaC03 and NH4MgP04, the vacuum device is operated to subject the processed liquid biomass to a vacuum of 20 to 200 mbar until a pH of at least 8, preferably at least 8.5.

By subjecting processed liquid biomass to a vacuum not only the dissolved methane is extracted, thereby increasing the yield of the present apparatus, but also dissolved C02. By extracting C02, the pH of the processed liquid biomass increases to the indicated range and, as a result, phosphor and magnesium in the form of NH4MgP04 and calcium in the form of CaC03 precipitates and, accordingly, can be conveniently removed from the process stream as a high-grade product.

NH4MgP04 and CaC03 removed can be brought to a substantially neutral pH, for example by using the S04- from the effluent gas conversion reactor (5), yielding a directly marketable product.

As indicated, the present apparatus is substantially kept in homeostasis, after start-up, for the indicated process conditions by microorganisms. However, for example depending on the type of biomass supplied to the apparatus, process conditions can deviate from the indicated conditions, for example, the pH in the acidification reactor can vary depending on the nitrogen content of the biomass supplied.

Accordingly, according to a preferred embodiment, the present apparatus comprises a controlling device to monitor the indicated pHs, temperatures and/or the redox potentials, and preferably, further comprises reactors, where appropriate, provided with heating devices for maintaining the temperature in the defined range, with pH regulating devices for maintaining the pHs in the defined range, and redox potential regulating devices for maintaining the redox potential in the defined range.

Temperature regulating devices can be heaters providing heat generated or derived from the apparatus itself, or heat from an external source, coolers providing cooling generated or derived from the apparatus itself, or cooling from an external source.

pH regulating devices can be holders comprising sugar, buffer, acid or basic liquid fitted with supply means for introducing the sugar, buffer, acid or basic liquid in the appropriate reactor, and/or a transport system controlling the flow of basic or acidic fluids in the apparatus itself, for example the leachate produced by the composting reactor (6).

Redox potential regulating devices can be holders comprising liquids with a defined redox potential fitted with supply means for introducing the liquids in the appropriate reactor.

According to still another preferred embodiment, the present one or more communicating inlets and outlets of the reactors comprise devices for isolation of microorganisms and for reintroducing the isolated microorganisms in the reactors from which they were derived from.

In other words, the microorganisms in a reactor inherently discharged with the process flows are continuously reintroduced into the reactor thereby providing a stable culture of microorganisms in the reactor, and, accordingly, a stable control of methane synthesis and other microbial processes.

The present apparatus is particularly suitable to process biomass, especially to convert biomass into methane and/or fertilizer, selected from the group consisting of liquid manure, manure, sewage sludge, domestic vegetable waste, agricultural plant residue, domestic plant residue, and combinations thereof.

As indicated above, methane and other high-grade products can be conveniently collected at the outlets of reactors comprised in the present apparatus. Accordingly, according to a preferred embodiment, the present invention relates to an apparatus wherein the methane is collected at the outlets of the methane synthesis reactor (2) and the methane synthesis reactor (3) and the fertilizer at the outlets of the nitrification reactor (4) and/or the composting reactor (6).

The apparatus as described above provides an efficient conversion of (waste) biomass into valuable products. Therefore, according to another aspect, the present invention relates to a method for conversion of biomass comprising:
a) supplying the acidification reactor (1) of the present apparatus with biomass;
b) operating:
   1) the acidification reactor (1) at a temperature of 45°C to 70°C and a pH of 3 to 4.5;
   2) the methane synthesis reactor (2) at a temperature of 20°C to 60°C, a pH of 6.5 to 8 and a redox potential of -150mV to -450mV under anaerobic conditions;
   3) the methane synthesis reactor (3) at a temperature of 20°C to 60°C, a pH of 6.5
   4) -450mV under anaerobic conditions;
   5) optionally, the effluent gas conversion reactor (5) at a temperature of 15°C to 35°C, a pH of 3.0 to 4.5 and a redox potential of more than 50 mV under aerobic conditions;
   6) the nitrification reactor (4) at a temperature of 15°C to 37°C, a pH of 6.5 to 7.5 and a redox potential of more than 50 mV under aerobic conditions;
wherein acidified biomass is transported from the acidification reactor (1) to the methane synthesis reactor (2), acidified liquid biomass is transported from the methane synthesis reactor (2) to the methane synthesis reactor (3), H₂S comprising gaseous effluent is transported from the acidification reactor (1) to the effluent gas conversion reactor (5), processed liquid biomass is transported from the methane synthesis reactor (3) to the nitrification reactor (4), wherein processed sludge biomass from the methane synthesis reactor (2) is transported to the composting reactor (6) operated at a temperature of 45°C to 75°C, a pH and an oxygen concentration of 2 to 20%, and wherein acetate comprising leachate is transported from the composting reactor (6) to the acidification reactor (1).

According to still another preferred embodiment of the present method, the biomass is selected from the group consisting of liquid manure, manure, sewage sludge, domestic vegetable waste, agriculture plant residue, domestic plant residue, and combinations thereof.

According to a further preferred embodiment of the present method, the conversion of biomass comprises conversion of biomass into methane and/or fertilizer.

## Claims

1. Apparatus for conversion of biomass, the apparatus comprises:
a) an acidification reactor (1) of a mixed fluid type reactor for microbial hydrolysis and acidification of biomass comprising at least one intake comprising at least one inlet for receiving biomass, at least one discharge end comprising at least one outlet for discharging acidified biomass, the acidification reactor (1) is configured to be operated at a temperature of 45°C to 70°C and a pH of 3 to 4.5;
b) a methane synthesis reactor (2) of a solid bed reactor type for the anaerobic microbial conversion of acidified biomass into methane comprising at least one intake for receiving acidified biomass from the acidification reactor (1), at least one discharge end comprising at least three outlets for discharging processed sludge biomass, methane comprising gaseous effluent and acidified liquid biomass, the methane synthesis reactor (2) is configured to be operated at a temperature of 20°C to 60°C, a pH of 6.5 to 8 and a redox potential of -150 mV to -450 mV;
c) a methane synthesis reactor (3) of a mixed fluid type reactor for anaerobic microbial conversion of acidified liquid biomass into methane comprising at least one intake comprising at least one inlet for receiving acidified liquid biomass from the methane synthesis reactor (2) and at least one discharge end comprising at least two outlets for discharging processed liquid biomass and methane comprising gaseous effluent, the methane synthesis reactor (3) is configured to be operated at a temperature of 20°C to 60°C, a pH of 6.5 to 8 and a redox potential of -150mV to -450mV;
d) a nitrification reactor (4) for aerobic microbial conversion of NH₄⁺ into N0₃⁻ comprising at least one intake comprising at least one inlet for receiving processed liquid biomass from the methane synthesis reactor (3), at least one discharge end comprising at least one outlet for discharging nitrified processed liquid biomass, the nitrification reactor is configured to be operated at a temperature of 15°C to 37°C, a pH of 6.5 to 7.5 and a redox potential of more than 50 mV, **characterized in that**
f) the apparatus further comprises:
a composting reactor (6) for microbial decomposition of processed sludge biomass comprising at least one intake comprising at least one inlet for receiving processed sludge biomass from the methane synthesis reactor (2) and at least one discharge end comprising at least one outlet for discharging composted biomass, the composting reactor (6) is configured to be operated at a temperature of 45°C to 75°C, a pH and an oxygen concentration of 2 to 20%, wherein the composting reactor (6) comprises a further outlet for discharging acetate comprising leachate and the acidification reactor (1) comprises a further inlet for receiving the acetate comprising leachate from the composting reactor (6).

2. Apparatus for conversion of biomass according to claim 1, wherein the acidification reactor (1) further comprises an outlet for discharging H₂S comprising gaseous effluent and the apparatus further comprises:
e) an effluent gas conversion reactor (5) for aerobic microbial conversion of H₂S into S0₄²⁻ comprising at least one intake comprising at least inlet for receiving H₂S comprising gaseous effluent from the acidification reactor (1) and at least one discharge end comprising at least one outlet for discharging C0₂, H₂0, S0₄²⁻, the effluent gas conversion reactor (5) is configured to be operated at a temperature of 15°C to 35°C, a pH of 3.0 to 4.5 and a redox potential of more than 50 mV.

3. Apparatus for conversion of biomass according to claim 1 or 2, wherein the methane synthesis reactor (2) comprises a phase separation device for separating the acidified biomass into an acidified liquid biomass and an acidified sludge biomass comprising at least one inlet for receiving acidified biomass and at least two outlets for discharging acidified liquid biomass to the methane synthesis reactor (3) and acidified sludge biomass, and a methane synthesis device comprising at least one inlet for receiving the acidified sludge biomass from the phase separation device and at least one outlet for discharging processed sludge biomass, the methane synthesis device is configured to be operated at a temperature of 20°C to 60°C, a pH of 6.5 to 8 and a redox potential of -150mV to -450mV.

4. Apparatus for conversion of biomass according to any of the claims 1 to 3, further comprising between the methane synthesis reactor (3) and the nitrification reactor (4) a vacuum device for concentrating the processed liquid biomass, the vacuum device comprises an inlet for receiving processed liquid biomass from the methane synthesis reactor (3) and an outlet for discharging concentrated processed liquid biomass to the nitrification reactor (4), an outlet for discharging C0₂ and methane and an outlet for discharging CaC0₃ and NH₄MgPO₄, the vacuum device is configured to be operated to subject the processed liquid biomass to a vacuum of 20 to 200 mbar until a pH of at least 8, preferably at least 8.5.

5. Apparatus for conversion of biomass according to any of the claims 1 to 4 further comprising a controlling device to monitor the pHs, the temperatures and/or the redox potentials as defined in any of the claims 1 to 4.

6. Apparatus for conversion of biomass according to any of the claims 1 to 5, further wherein the reactors (1) to (5) are provided with heating devices for maintaining the temperature in the defined range, with pH regulating devices for maintaining the pHs in the defined range, and reactors (2) to (5) are provided with redox potential regulating devices for maintaining the redox potential in the defined range.

7. Apparatus for conversion of biomass according to any of the claims 1 to 6 wherein one or more of the communicating inlets and outlets of the reactors comprise devices for isolation of microorganisms and reintroduction of the isolated microorganisms in the reactors from which they were derived from.

8. Apparatus for conversion of biomass according to any one of the claims 1 to 7 wherein conversion of biomass comprises conversion of biomass into methane and/or fertilizer, wherein the methane is collected at the outlets of the methane synthesis reactor (2) and the methane synthesis reactor (3) and the fertilizer at the outlets of the nitrification reactor (4) and/or the composting reactor (6).

9. Method for conversion of biomass in an apparatus according to any of claims 1 to 8 comprising:
a) supplying an acidification reactor (1) with biomass;
b) operating:
1. the acidification reactor (1) at a temperature of 45°C to 70°C and a pH of 3 to 4.5;
2. the methane synthesis reactor (2) at a temperature of 20°C to 60°C, a pH of 6.5 to 8 and a redox potential of -150mV to -450mV under anaerobic conditions;
3. the methane synthesis reactor (3) at a temperature of 20°C to 60°C, a pH of 6.5 to 8 and a redox potential of -150mV to -450mV under anaerobic conditions;
4. optionally, the effluent gas conversion reactor (5) at a temperature of 15°C to 35°C, a pH of 3.0 to 4.5 and a redox potential of more than 50 mV under aerobic conditions;
5. the nitrification reactor (4) at a temperature of 15°C to 37°C, a pH of 6.5 to 7.5 and a redox potential of more than 50 mV under aerobic conditions; wherein acidified biomass is transported from the acidification reactor (1) to the methane synthesis reactor (2), acidified liquid biomass is transported from the methane synthesis reactor (2) to the methane synthesis reactor (3), H₂S comprising gaseous effluent is transported from the acidification reactor (1) to the effluent gas conversion reactor (5), processed liquid biomass is transported from the methane synthesis reactor (3) to the nitrification reactor (4), wherein processed sludge biomass from the methane synthesis reactor (2) is transported to the composting reactor (6) operated at a temperature of 45°C to 75°C, a pH and an oxygen concentration of 2 to 20%, and wherein acetate comprising leachate is transported from the composting reactor (6) to the acidification reactor (1).

10. Method for conversion of biomass according to claim 9, wherein the biomass is selected from the group consisting of liquid manure, manure, sewage sludge, domestic vegetable waste, agricultural plant residue, domestic plant residue, and combinations thereof.

11. Method for conversion of biomass according to claim 9 or 10, wherein conversion of biomass comprises conversion of biomass into methane and/or fertilizer.

## Patentansprüche

1. Anlage zum Umwandeln von Biomasse, wobei die Anlage umfasst:
a) einen Versäuerungsreaktor (1) vom Typ eines Mischfluidreaktors für mikrobielle Hydrolyse und Versäuerung von Biomasse, umfassend wenigstens eine Aufnahme umfassend wenigstens einen Einlass zum Aufnehmen von Biomasse, wenigstens ein Ausstoßende umfassend wenigstens einen Auslass zum Ausstoßen von versäuerter Biomasse, wobei der Versäuerungsreaktor (1) dafür gestaltet ist, bei einer Temperatur von 45 °C bis 70 °C und einem pH-Wert von 3 bis 4,5 betrieben zu werden;
b) einen Methansynthesereaktor (2) vom Typ eines Festbettreaktors für die anaerobe mikrobielle Umwandlung von versäuerter Biomasse zu Methan, umfassend wenigstens eine Aufnahme zum Aufnehmen von versäuerter Biomasse von dem Versäuerungsreaktor (1), wenigstens ein Ausstoßende umfassend wenigstens drei Auslässe zum Ausstoßen von verarbeitetem Biomasseschlamm, methanhaltigem gasförmigem Abstrom und versäuerter flüssiger Biomasse, wobei der Methansynthesereaktor (2) dafür gestaltet ist, bei einer Temperatur von 20 °C bis 60 °C, einem pH-Wert von 6,5 bis 8 und einem Redoxpotential von -150 mV bis -450 mV betrieben zu werden;
c) einen Methansynthesereaktor (3) vom Typ eines Mischfluidreaktors für die anaerobe mikrobielle Umwandlung von versäuerter flüssiger Biomasse zu Methan, umfassend wenigstens eine Aufnahme umfassend wenigstens einen Einlass zum Aufnehmen von versäuerter flüssiger Biomasse von dem Methansynthesereaktor (2) und wenigstens ein Ausstoßende umfassend wenigstens zwei Auslässe zum Ausstoßen von verarbeiteter flüssiger Biomasse und methanhaltigem gasförmigem Abstrom, wobei der Methansynthesereaktor (3) dafür gestaltet ist, bei einer Temperatur von 20 °C bis 60 °C, einem pH-Wert von 6,5 bis 8 und einem Redoxpotential von -150 mV bis -450 mV betrieben zu werden;
d) einen Nitrifikationsreaktor (4) für die aerobe mikrobielle Umwandlung von NH₄⁺ zu NO₃⁻, umfassend wenigstens eine Aufnahme umfassend wenigstens einen Einlass zum Aufnehmen von verarbeiteter flüssiger Biomasse von dem Methansynthesereaktor (3), wenigstens ein Ausstoßende umfassend wenigstens einen Auslass zum Ausstoßen von nitrifizierter verarbeiteter flüssiger Biomasse, wobei der Nitrifikationsreaktor dafür gestaltet ist, bei einer Temperatur von 15 °C bis 37 °C, einem pH-Wert von 6,5 bis 7,5 und einem Redoxpotential von mehr als 50 mV betrieben zu werden, **dadurch gekennzeichnet, dass**
f) die Anlage ferner umfasst: einen Kompostierungsreaktor (6) für die mikrobielle Zersetzung von verarbeitetem Biomasseschlamm, umfassend wenigstens eine Aufnahme umfassend wenigstens einen Einlass zum Aufnehmen von verarbeitetem Biomasseschlamm von dem Methansynthesereaktor (2) und wenigstens ein Ausstoßende umfassend wenigstens einen Auslass zum Ausstoßen von kompostierter Biomasse, wobei der Kompostierungsreaktor (6) dafür gestaltet ist, bei einer Temperatur von 45 °C bis 75 °C, einem pH-Wert und einer Sauerstoffkonzentration von 2 bis 20 % betrieben zu werden, wobei der Kompostierungsreaktor (6) einen weiteren Auslass zum Ausstoßen von acetathaltiger Laugungslösung umfasst und der Versäuerungsreaktor (1) einen weiteren Einlass zum Aufnehmen der acetathaltigen Laugungslösung von dem Kompostierungsreaktor (6) umfasst.

2. Anlage zum Umwandeln von Biomasse gemäß Anspruch 1, wobei der Versäuerungsreaktor (1) ferner einen Auslass zum Ausstoßen von H₂S-umfassendem gasförmigem Abstrom umfasst und die Anlage ferner umfasst:
e) einen Abgas-Umwandlungsreaktor (5) für die aerobe mikrobielle Umwandlung von H₂S zu SO₄²⁻, umfassend wenigstens eine Aufnahme umfassend wenigstens einen Einlass zum Aufnehmen von H₂S-umfassendem gasförmigem Abstrom von dem Versäuerungsreaktor (1) und wenigstens ein Ausstoßende umfassend wenigstens einen Auslass zum Ausstoßen von CO₂, H₂O, SO₄²⁻, wobei der Abgas-Umwandlungsreaktor (5) dafür gestaltet ist, bei einer Temperatur von 15 °C bis 35 °C, einem pH-Wert von 3,0 bis 4,5 und einem Redoxpotential von mehr als 50 mV betrieben zu werden.

3. Anlage zum Umwandeln von Biomasse gemäß Anspruch 1 oder 2, wobei der Methansynthesereaktor (2) eine Phasentrennungsvorrichtung zum Auftrennen der versäuerten Biomasse in eine versäuerte flüssige Biomasse und einen versäuerten Biomasseschlamm umfasst, umfassend wenigstens einen Einlass zum Aufnehmen von versäuerter Biomasse und wenigstens zwei Auslässe zum Ausstoßen von versäuerter flüssiger Biomasse zu dem Methansynthesereaktor (3) und von versäuertem Biomasseschlamm, und eine Methansynthesevorrichtung umfassend wenigstens einen Einlass zum Aufnehmen des versäuerten Biomasseschlamms von der Phasentrennungsvorrichtung und wenigstens einen Auslass zum Ausstoßen von verarbeitetem Biomasseschlamm, wobei die Methansynthesevorrichtung dafür gestaltet ist, bei einer Temperatur von 20 °C bis 60 °C, einem pH-Wert von 6,5 bis 8 und einem Redoxpotential von - 150 mV bis -450 mV betrieben zu werden.

4. Anlage zum Umwandeln von Biomasse gemäß einem der Ansprüche 1 bis 3, ferner umfassend zwischen dem Methansynthesereaktor (3) und dem Nitrifikationsreaktor (4) eine Unterdruckvorrichtung zum Konzentrieren der verarbeiteten flüssigen Biomasse, wobei die Unterdruckvorrichtung einen Einlass zum Aufnehmen von verarbeiteter flüssiger Biomasse von dem Methansynthesereaktor (3) und einen Auslass zum Ausstoßen von konzentrierter verarbeiteter flüssiger Biomasse zu dem Nitrifikationsreaktor (4), einen Auslass zum Ausstoßen von CO₂ und Methan und einen Auslass zum Ausstoßen von CaCO₃ und NH₄MgPO₄ umfasst, wobei die Unterdruckvorrichtung dafür gestaltet ist, betrieben zu werden, um die verarbeitete flüssige Biomasse einem Unterdruck von 20 bis 200 mbar bis zu einem pH-Wert von wenigstens 8, vorzugsweise wenigstens 8,5, zu unterwerfen.

5. Anlage zum Umwandeln von Biomasse gemäß einem der Ansprüche 1 bis 4, ferner umfassend eine Steuervorrichtung zum Überwachen der pH-Werte, der Temperaturen und/oder der Redoxpotentiale gemäß einem der Ansprüche 1 bis 4.

6. Anlage zum Umwandeln von Biomasse gemäß einem der Ansprüche 1 bis 5, wobei ferner die Reaktoren (1) bis (5) mit Heizvorrichtungen zum Halten der Temperatur in dem definierten Bereich, mit pH-Regulationsvorrichtungen zum Halten der pH-Werte in dem definierten Bereich ausgestattet sind und die Reaktoren (2) bis (5) mit Redoxpotential-Regulationsvorrichtungen zum Halten des Redoxpotentials in dem definierten Bereich ausgestattet sind.

7. Anlage zum Umwandeln von Biomasse gemäß einem der Ansprüche 1 bis 6, wobei eine oder mehrere der verbundenen Einlässe und Auslässe der Reaktoren Vorrichtungen zum Isolieren von Mikroorganismen und Rückführen der isolierten Mikroorganismen in die Reaktoren, aus denen sie erhalten wurden, umfassen.

8. Anlage zum Umwandeln von Biomasse gemäß einem der Ansprüche 1 bis 7, wobei die Umwandlung von Biomasse Umwandlung von Biomasse zu Methan und/oder Düngemittel umfasst, wobei das Methan an den Auslässen des Methansynthesereaktors (2) und des Methansynthesereaktors (3) gesammelt wird und das Düngemittel an den Auslässen des Nitrifikationsreaktors (4) und/oder des Kompostierungsreaktors (6) gesammelt wird.

9. Verfahren zum Umwandeln von Biomasse in einer Anlage gemäß einem der Ansprüche 1 bis 8, umfassend:
a) Zuführen von Biomasse zu einem Versäuerungsreaktor (1);
b) Betreiben:
1. des Versäuerungsreaktors (1) bei einer Temperatur von 45 °C bis 70 °C und einem pH-Wert von 3 bis 4,5;
2. des Methansynthesereaktors (2) bei einer Temperatur von 20 °C bis 60 °C, einem pH-Wert von 6,5 bis 8 und einem Redoxpotential von -150 mV bis -450 mV unter anaeroben Bedingungen;
3. des Methansynthesereaktors (3) bei einer Temperatur von 20 °C bis 60 °C, einem pH-Wert von 6,5 bis 8 und einem Redoxpotential von -150 mV bis -450 mV unter anaeroben Bedingungen;
4. gegebenenfalls des Abgas-Umwandlungsreaktors (5) bei einer Temperatur von 15 °C bis 35 °C, einem pH-Wert von 3,0 bis 4,5 und einem Redoxpotential von mehr als 50 mV unter aeroben Bedingungen;
5. des Nitrifikationsreaktors (4) bei einer Temperatur von 15 °C bis 37 °C, einem pH-Wert von 6,5 bis 7,5 und einem Redoxpotential von mehr als 50 mV unter aeroben Bedingungen;
wobei versäuerte Biomasse von dem Versäuerungsreaktor (1) zu dem Methansynthesereaktor (2) befördert wird, versäuerte flüssige Biomasse von dem Methansynthesereaktor (2) zu dem Methansynthesereaktor (3) befördert wird, H₂S-umfassender gasförmiger Abstrom von dem Versäuerungsreaktor (1) zu dem Abgas-Umwandlungsreaktor (5) befördert wird, verarbeitete flüssige Biomasse von dem Methansynthesereaktor (3) zu dem Nitrifikationsreaktor (4) befördert wird, wobei verarbeiteter Biomasseschlamm von dem Methansynthesereaktor (2) zu dem Kompostierungsreaktor (6) befördert wird, der bei einer Temperatur von 45 °C bis 75 °C, einem pH-Wert und einer Sauerstoffkonzentration von 2 bis 20 % betrieben wird, und wobei acetathaltige Laugungslösung von dem Kompostierungsreaktor (6) zu dem Versäuerungsreaktor (1) befördert wird.

10. Verfahren zum Umwandeln von Biomasse gemäß Anspruch 9, wobei die Biomasse ausgewählt ist aus der Gruppe bestehend aus flüssiger Gülle, Gülle, Klärschlamm, pflanzlichem Haushaltsabfall, landwirtschaftlichen Pflanzenrückständen, Haushalts-Pflanzenrückständen und Kombinationen davon.

11. Verfahren zum Umwandeln von Biomasse gemäß Anspruch 9 oder 10, wobei Umwandeln von Biomasse Umwandeln von Biomasse zu Methan und/oder Düngemittel umfasst.

## Revendications

1. Appareil de conversion de biomasse, l'appareil comprenant :
a) un réacteur d'acidification (1) d'un réacteur de type à fluide mixte pour l'hydrolyse microbienne et l'acidification de biomasse comprenant au moins un orifice d'admission comprenant au moins un orifice d'entrée pour recevoir la biomasse, au moins une extrémité d'évacuation comprenant au moins un orifice de sortie pour évacuer la biomasse acidifiée, le réacteur d'acidification (1) est configuré pour être exploité à une température de 45 °C à 70 °C et un pH de 3 à 4,5 ;
b) un réacteur de synthèse de méthane (2) d'un type de réacteur à lit solide pour la conversion microbienne anaérobie de biomasse acidifiée en méthane comprenant au moins un orifice d'admission pour recevoir la biomasse acidifiée provenant du réacteur d'acidification (1), au moins une extrémité d'évacuation comprenant au moins trois orifices de sortie pour évacuer la biomasse en boue traitée, le méthane comprenant un effluent gazeux et la biomasse liquide acidifiée, le réacteur de synthèse de méthane (2) est configuré pour être exploité à une température de 20 °C à 60 °C, un pH de 6,5 à 8 et un potentiel d'oxydoréduction de -150 mV à -450 mV ;
c) un réacteur de synthèse de méthane (3) d'un réacteur de type à fluide mixte pour la conversion microbienne anaérobie de biomasse liquide acidifiée en méthane comprenant au moins un orifice d'admission comprenant au moins un orifice d'entrée pour recevoir la biomasse liquide acidifiée provenant du réacteur de synthèse de méthane (2) et au moins une extrémité d'évacuation comprenant au moins deux orifices de sortie pour évacuer la biomasse liquide traitée et le méthane comprenant un effluent gazeux, le réacteur de synthèse de méthane (3) est configuré pour être exploité à une température de 20 °C à 60 °C, un pH de 6,5 à 8 et un potentiel d'oxydoréduction de -150 mV à -450 mV ;
d) un réacteur de nitrification (4) pour la conversion microbienne aérobie de NH₄⁺ + en NO₃⁻ comprenant au moins un orifice d'admission comprenant au moins un orifice d'entrée pour recevoir la biomasse liquide traitée provenant du réacteur de synthèse de méthane (3), au moins une extrémité d'évacuation comprenant au moins un orifice de sortie pour évacuer la biomasse liquide traitée et nitrifiée, le réacteur de nitrification est configuré pour être exploité à une température de 15 °C à 37 °C, un pH de 6,5 à 7,5 et un potentiel d'oxydoréduction de plus de 50 mV, **caractérisé en ce que**
f) l'appareil comprend en outre :
un réacteur de compostage (6) pour la décomposition microbienne de la biomasse en boue traitée comprenant au moins un orifice d'admission comprenant au moins un orifice d'entrée pour recevoir la biomasse en boue traitée provenant du réacteur de synthèse de méthane (2) et au moins une extrémité d'évacuation comprenant au moins un orifice de sortie pour évacuer la biomasse compostée, le réacteur de compostage (6) est configuré pour être exploité à une température de 45 °C à 75 °C, un pH et une concentration en oxygène de 2 à 20 %, dans lequel le réacteur de compostage (6) comprend un orifice de sortie supplémentaire pour évacuer l'acétate comprenant un lixiviat et le réacteur d'acidification (1) comprend un orifice d'entrée supplémentaire pour recevoir l'acétate comprenant un lixiviat provenant du réacteur de compostage (6).

2. Appareil de conversion de biomasse selon la revendication 1, dans lequel le réacteur d'acidification (1) comprend en outre un orifice de sortie pour évacuer le H₂S comprenant un effluent gazeux et l'appareil comprend en outre :
e) un réacteur de conversion de gaz effluent (5) pour la conversion microbienne aérobie de H₂S en SO₄²⁻ comprenant au moins un orifice d'admission comprenant au moins un orifice d'entrée pour recevoir le H₂S comprenant un effluent gazeux provenant du réacteur d'acidification (1) et au moins une extrémité d'évacuation comprenant au moins un orifice de sortie pour évacuer le CO₂, le H₂O, le SO₄²⁻, le réacteur de conversion de gaz effluent (5) est configuré pour être exploité à une température de 15 °C à 35 °C, un pH de 3,0 à 4,5 et un potentiel d'oxydoréduction de plus de 50 mV.

3. Appareil de conversion de biomasse selon la revendication 1 ou 2, dans lequel le réacteur de synthèse de méthane (2) comprend un dispositif de séparation de phase pour séparer la biomasse acidifiée en une biomasse liquide acidifiée et une biomasse en boue acidifiée comprenant au moins un orifice d'entrée pour recevoir la biomasse acidifiée et au moins deux orifices de sortie pour évacuer la biomasse liquide acidifiée dans le réacteur de synthèse de méthane (3) et la biomasse en boue acidifiée, et un dispositif de synthèse de méthane comprenant au moins un orifice d'entrée pour recevoir la biomasse en boue acidifiée provenant du dispositif de séparation de phase et au moins un orifice de sortie pour évacuer la biomasse en boue traitée, le dispositif de synthèse de méthane est configuré pour être exploité à une température de 20 °C à 60 °C, un pH de 6,5 à 8 et un potentiel d'oxydoréduction de -150 mV à -450 mV.

4. Appareil de conversion de biomasse selon l'une quelconque des revendications 1 à 3, comprenant en outre entre le réacteur de synthèse de méthane (3) et le réacteur de nitrification (4), un dispositif de vide pour concentrer la biomasse liquide traitée, le dispositif de vide comprend un orifice d'entrée pour recevoir la biomasse liquide traitée provenant du réacteur de synthèse de méthane (3) et un orifice de sortie pour évacuer la biomasse liquide traitée concentrée dans le réacteur de nitrification (4), un orifice de sortie pour évacuer le CO₂ et le méthane et un orifice de sortie pour évacuer le CaCO₃ et le NH₄MgPO₄, le dispositif de vide est configuré pour être exploité pour soumettre la biomasse liquide traitée à un vide de 20 à 200 mbar jusqu'à un pH d'au moins 8, de préférence d'au moins 8,5.

5. Appareil de conversion de biomasse selon l'une quelconque des revendications 1 à 4, comprenant en outre un dispositif de commande pour surveiller les pH, les températures et/ou les potentiels d'oxydoréduction tels que définis dans l'une quelconque des revendications 1 à 4.

6. Appareil de conversion de biomasse selon l'une quelconque des revendications 1 à 5, en outre dans lequel les réacteurs (1) à (5) sont pourvus de dispositifs de chauffage pour maintenir la température dans la plage définie, de dispositifs de régulation de pH pour maintenir les pH dans la plage définie, et les réacteurs (2) à (5) sont pourvus de dispositifs de régulation de potentiel d'oxydoréduction pour maintenir le potentiel d'oxydoréduction dans la plage définie.

7. Appareil de conversion de biomasse selon l'une quelconque des revendications 1 à 6, dans lequel un ou plusieurs des orifices d'entrée et des orifices de sortie de communication des réacteurs comprennent des dispositifs d'isolement de micro-organismes et de réintroduction des micro-organismes isolés dans les réacteurs dont ils ont été dérivés.

8. Appareil de conversion de biomasse selon l'une quelconque des revendications 1 à 7, dans lequel la conversion de biomasse comprend la conversion de biomasse en méthane et/ou en fertilisant, dans lequel le méthane est recueilli au niveau des orifices de sortie du réacteur de synthèse de méthane (2) et du réacteur de synthèse de méthane (3) et le fertilisant au niveau des orifices de sortie du réacteur de nitrification (4) et/ou du réacteur de compostage (6).

9. Procédé de conversion de biomasse dans un appareil selon l'une quelconque des revendications 1 à 8, comprenant :
a) l'alimentation d'un réacteur d'acidification (1) en biomasse ;
b) l'exploitation:
1. du réacteur d'acidification (1) à une température de 45 °C à 70 °C et un pH de 3 à 4,5 ;
2. du réacteur de synthèse de méthane (2) à une température de 20 °C à 60 °C, un pH de 6, 5 à 8 et un potentiel d'oxydoréduction de -150 mV à -450 mV dans des conditions anaérobies ;
3. du réacteur de synthèse de méthane (3) à une température de 20 °C à 60 °C, un pH de 6, 5 à 8 et un potentiel d'oxydoréduction de -150 mV à -450 mV dans des conditions anaérobies ;
4. facultativement, du réacteur de conversion de gaz effluent (5) à une température de 15 °C à 35 °C, un pH de 3,0 à 4,5 et un potentiel d'oxydoréduction de plus de 50 mV dans des conditions aérobies ;
5. du réacteur de nitrification (4) à une température de 15 °C à 37 °C, un pH de 6,5 à 7,5 et un potentiel d'oxydoréduction de plus de 50 mV dans des conditions aérobies ;
dans lequel la biomasse acidifiée est transportée du réacteur d'acidification (1) au réacteur de synthèse de méthane (2), la biomasse liquide acidifiée est transportée du réacteur de synthèse de méthane (2) au réacteur de synthèse de méthane (3), le H₂S comprenant un effluent gazeux est transporté du réacteur d'acidification (1) au réacteur de conversion de gaz effluent (5), la biomasse liquide traitée est transportée du réacteur de synthèse de méthane (3) au réacteur de nitrification (4), dans lequel la biomasse en boue traitée provenant du réacteur de synthèse de méthane (2) est transportée vers le réacteur de compostage (6) exploité à une température de 45 °C à 75 °C, un pH et une concentration en oxygène de 2 à 20 %, et dans lequel l'acétate comprenant un lixiviat est transporté du réacteur de compostage (6) au réacteur d'acidification (1).

10. Procédé de conversion de biomasse selon la revendication 9, dans lequel la biomasse est choisie dans le groupe constitué du purin, du fumier, de boues d'épuration, de déchets de légumes domestiques, de résidus végétaux agricoles, de résidus végétaux domestiques, et de leurs combinaisons.

11. Procédé de conversion de biomasse selon la revendication 9 ou 10, dans lequel la conversion de biomasse comprend la conversion de biomasse en méthane et/ou en fertilisant.
